# EUROPÄISCHE PATENTSCHRIFT

(11) **EP 0 857 042 B1**
(45) Veröffentlichungstag und Bekanntmachung des Hinweises auf die Patenterteilung: **07.11.2001**
(21) Anmeldenummer: 95934031.6
(22) Anmeldetag: 20.10.1995
(51) Int. Cl.: A61F 2/44

(54) **ZWISCHENWIRBEL-IMPLANTAT MIT KOMPRIMIERBAREM HOHLKÖRPERPROFIL**
INTERVERTEBRAL IMPLANT WITH COMPRESSIBLE SHAPED HOLLOW ELEMENT
IMPLANT INTERVERTEBRAL AVEC CORPS CREUX PROFILE ET COMPRESSIBLE

(43) Veröffentlichungstag der Anmeldung: 12.08.1998
(73) Patentinhaber: SYNTHES AG, Chur, 7002 Chur (CH)
(72) Erfinder: KNOTHE, Inga, CH-2503 Biel (CH); BENOIT, Alfred, CH-2543 Lengnau (CH)
(74) Vertreter: Lusuardi, Werther Giovanni, Dr.
(86) Internationale Anmeldenummer: CH9500244
(87) Internationale Veröffentlichungsnummer: WO9715247

(56) Entgegenhaltungen:
- EP-A- 0 260 044
- EP-A- 0 664 994
- DE-A- 4 409 392
- DE-C- 4 012 622

## Beschreibung

Die Erfindung betrifft ein Zwischenwirbel-Implantat gemäss dem Oberbegriff des Patentanspruchs 1.
Solche Zwischenwirbel-Implantate werden bei der Fusion von Wirbelkörpern eingesetzt, insbesondere im Bereich der lumbalen Wirbelsäule. Pro Zwischenwirbelraum werden ein bis zwei Implantate verwendet.
Aus dem Stand der Technik sind bereits verschiedene Typen derartiger Zwischenwirbel-Implantate bekannt, welche allerdings folgende Nachteile aufweisen:
- um das Implantat in den Zwischenwirbelbereich einführen zu können müssen die betroffenen Wirbel mit geeigneten Instrumenten distrahiert werden; und
- es besteht die Gefahr des Einsinkens des Implantats in die Endplatten der betroffenen Wirbel.
Aus der DE-C-4 012 622 ist ein Wirbelkörperimplantat gemäss dem Oberbegriff des Anspruchs 1 bekannt, welches zweiteilig ausgebildet ist. Der äussere Teil ist elastisch ausgebildet, um den inneren Teil aufnehmen und darin arretieren zu können.
Dieses bekannte Implantat weist folgende Nachteile auf:
- Zweiteiligkeit statt Einteiligkeit;
- nach erfolgtem Zusammenbau der beiden Teile ist kein Platz mehr im Inneren vorhanden um Knochenersatzmaterial aufzunehmen;
- nach erfolgtem Zusammenbau der beiden Teile ist das Implantat zwingenderweise unelastisch.

Hier will die Erfindung Abhilfe schaffen. Der Erfindung liegt die Aufgabe zugrunde, ein Zwischenwirbel-Implantat zu schaffen, welches möglichst ohne Distraktionsinstrument in kontrollierbarer Weise in den ausgeräumten Zwischenwirbelraum einführbar ist.

Zur Lösung dieses Problems ist die eingangs genannte Anordnung durch die Merkmale des kennzeichnenden Teils des unabhängigen Anspruchs 1 weitergebildet.

Das abgeflachte Hohlkörperprofil kann die Form eines Hohlzylinders, hohlen Kegelstumpfs, hohlen Pyramidenstumpfs oder hohlen Keilstumpfes besitzen.

Dimensionen, Geometrie und Werkstoff sollen so gewählt werden, dass der maximale Abstand H zwischen oberer und unterer Knochenkontaktfläche um 0,5 - 5,0 mm , vorzugsweise um 1 - 3 mm reduzierbar ist.
Bei Anwendung einer Druckkraft von 12 Newton auf die obere und untere Knochenkontaktfläche sollte der maximale Abstand H um 15 % bis 30 %, vorzugsweise um 20 % bis 25 % reduzierbar sein.
Die Komprimierbarkeit des Implantates gestattet dessen Einführung ohne ein Distraktionsinstrument anwenden zu müssen.

Damit ist der Vorteil erzielbar, dass ein minimaler Kraftaufwand für die Implantation nötig ist und ein kontrolliertes Einfügen des komprimierten Implantates möglich ist. Eine minimalinvasive und offene Operationstechnik ist damit anwendbar.

Die grossen Auflageflächen des Implantates verhindern dessen Einsinken in die Endplatten.

Eine bevorzugte Weiterbildung der Erfindung besteht darin, dass im Innenraum des Hohlkörperprofils eine Einrastlippe vorgesehen ist, welche in eine Sperre einrastbar ist. Bei eingerasteter Einrastlippe ist die Komprimierbarkeit des Hohlkörperprofils wieder aufgehoben, so dass ein übliches rigides Implantat resultiert.
Knochenspäne oder Knochenersatzmaterialien können leicht in das vorne und hinten offene Hohlkörperprofil eingefüllt werden und das Implantat mit wenigen Handgriffen sicher montiert werden.

Um ein rasches Einwachsen des Knochens zu fördern, ist das Implantat zweckmässigerweise mit Perforationen, vorzugsweise in Form von Längsausnehmungen versehen. Die Längsausnehmungen erlauben eine Kontrolle des Knocheneinwachsens mittels Röntgenaufnahmen bei Verwendung eines röntgenstrahlenundurchlässigen Materials.

Zur Erhöhung der Lagestabilität des Implantates ist die obere und/oder untere Knochenkontaktfläche mit einer dreidimensionalen Strukturierung, vorzugsweise in Form von Längsrillen versehen.

Folgende weitere Vorteile des erfindungsgemässen Implantats ergeben sich gegenüber dem Stand der Technik:
- Verrutschsicherheit;
- verbesserte Röntgendurchlässigkeit; und
- erhöhte Elastizität des Implantats.

Die Erfindung und Weiterbildungen der Erfindung werden im folgenden anhand der teilweise schematischen Darstellungen eines Ausführungsbeispiels noch näher erläutert.

Es zeigen:
Fig. 1 eine perspektivische Darstellung des erfindungsgemässen Implantats im komprimierbaren Zustand mit loser Einrastlippe; und
Fig. 2 einen Querschnitt durch das Implantats nach Fig. 1 im nicht-komprimierbaren Zustand mit eingerasteter Einrastlippe.

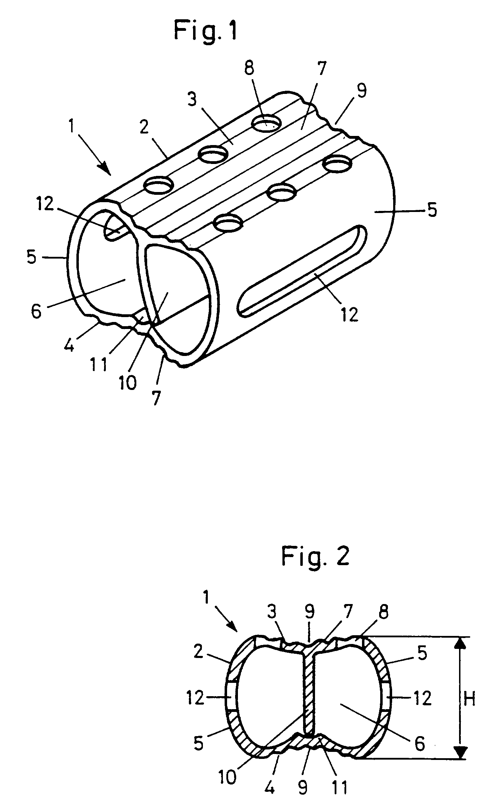

Das in den Fig. 1 und 2 dargestellte Zwischenwirbel-Implantat besteht im wesentlichen aus einem Hohlkörperprofil 1 in Form eines Hohlzylinders, dessen Mantelfläche 2 aus einer abgeflachten, oberen Knochenkontaktfläche 3, einer abgeflachten, unteren Knochenkontaktfläche 4 und zwei Seitenflächen 5 besteht.
Das Implantat kann aus einem geeigneten Metall (z.B. Titan), insbesondere aber auch aus einem körperverträglichen Kunststoff, wie Polyethylen gefertigt werden.
Die obere und untere Knochenkontaktfläche 3 und 4 ist gegen den Innenraum 6 des Hohlkörperprofils 1 elastisch komprimierbar, derart dass der maximale Abstand H zwischen oberer und unterer Knochenkontaktfläche 3 und 4 reduzierbar ist, typischerweise um 2 mm. Dies erlaubt eine einfachere Einführung des Implantats in den Zwischenwirbelraum.

Die Seitenflächen 5 sind mit Langlochausnehmungen 12 versehen, welche bei Verwendung eines Kunststoffmaterials auch weggelassen werden können. Die obere Knochenkontaktfläche 3 und/oder untere Knochenkontaktfläche 4 ist mit Perforationen 8 sowie einer Mittelrille 9 versehen.
Im Innenraum 6 des Hohlkörperprofils 1 ist eine Einrastlippe 10 vorgesehen, welche in eine Sperre 11 einrastbar ist.

Nachstehend wird nun die klinische Anwendung im Detail beschrieben. Das in Fig. 1 gezeigte Hohlkörperprofil 1 wird mittels eines geeigneten Kompressionswerkzeugs gefasst, so dass auf die obere und untere Knochenkontaktfläche 3 und 4 eine Druckkraft - typischerweise von 12 Newton - ausgeübt werden kann, welche das Hohlkörperprofil 1 komprimiert. In diesem Zustand wird das Implantat nun möglichst ohne Distraktionsinstrumente in den ausgeräumten Zwischenwirbelraum eingeführt, wobei die dreidimensionalen Strukturierungen 7, in Form von Längsrillen die Führung des Implantats erleichtern und eine Sicherung gegen ein allfälliges Verrutschen des Implantats bieten.
Das Kompressionswerkzeug wird entfernt und die Einrastlippe 10 aufgerichtet und in die Sperre 11 eingerastet, so dass das Hohlkörperprofil 1 nun nicht mehr komprimierbar ist. Das Implantat kann nun mit Knochenspänen oder Knochenersatzmaterial gefüllt werden.

## Patentansprüche

1. Zwischenwirbel-Implantat bestehend aus einem abgeflachten Hohlkörperprofil (1), dessen Mantelfläche (2) aus einer abgeflachten, oberen Knochenkontaktfläche (3), einer abgeflachten, unteren Knochenkontaktfläche (4) und einer Seitenfläche (5) besteht, wobei die obere und untere Knochenkontaktfläche (3;4) gegen den Innenraum (6) des Hohlkörperprofils (1) elastisch komprimierbar sind,
**dadurch gekennzeichnet, dass**
a) das Hohlkörperprofil (1) in Form eines Hohlzylinders, hohlen Kegelstumpfs, hohlen Pyramidenstumpfs oder hohlen Keilstumpfes ausgebildet ist;
b) die Mantelfläche (2) des Hohlkörperprofils (1) eine zweite Seitenfläche (5) aufweist; und
c) bei Anwendung einer Druckkraft die obere und untere Knochenkontaktfläche (3;4) derart gegeneinander komprimierbar sind, dass der maximale Abstand H zwischen oberer und unterer Knochenkontaktfläche (3;4) um 0,5 - 5,0 mm reduzierbar ist.

2. Implantat nach Anspruch 1, **dadurch gekennzeichnet, dass** der maximale Abstand H zwischen oberer und unterer Knochenkontaktfläche (3;4) um 1 - 3 mm reduzierbar ist.

3. Implantat nach Anspruch 1 oder 2, **dadurch gekennzeichnet, dass** der maximale Abstand H bei Anwendung einer Druckkraft von 12 Newton auf die obere und untere Knochenkontaktfläche (3;4) um 15 % bis 30 % reduzierbar ist.

4. Implantat nach Anspruch 3, **dadurch gekennzeichnet, dass** der maximale Abstand H bei Anwendung einer Druckkraft von 12 Newton auf die obere und untere Knochenkontaktfläche (3;4) um 20 % bis 25 % reduzierbar ist.

5. Implantat nach einem der Ansprüche 1 - 4, **dadurch gekennzeichnet, dass** im Innenraum (6) des Hohlkörperprofils (1) eine Einrastlippe (10) vorgesehen ist, welche, unter Aufhebung der Komprimierbarkeit des Hohlkörperprofils (1), in eine Sperre (11) einrastbar ist.

6. Implantat nach einem der Ansprüche 1 - 5, **dadurch gekennzeichnet, dass** die Seitenflächen (5) mit Langlochausnehmungen (12) versehen sind.

7. Implantat nach einem der Ansprüche 1 - 6, **dadurch gekennzeichnet, dass** die obere Knochenkontaktfläche (3) und/oder die untere Knochenkontaktfläche (4) mit einer dreidimensionalen Strukturierung (7) versehen ist.

8. Implantat nach Anspruch 7, **dadurch gekennzeichnet, dass** die dreidimensionale Strukturierung (7) in Form von Längsrillen realisiert ist.

9. Implantat nach einem der Ansprüche 1 - 8, **dadurch gekennzeichnet, dass** die obere Knochenkontaktfläche (3) und/oder die untere Knochenkontaktfläche (4) mit Perforationen (8) versehen ist.

10. Implantat nach einem der Ansprüche 1 - 9, **dadurch gekennzeichnet, dass** die obere Knochenkontaktfläche (3) und/oder die untere Knochenkontaktfläche (4) eine oder mehrere Mittelrillen (9) aufweisen.

11. Implantat nach einem der Ansprüche 1 - 10, **dadurch gekennzeichnet, dass** es aus einem biokompatiblen Kunststoff gefertigt ist.

12. Implantat nach Anspruch 11, **dadurch gekennzeichnet, dass** der biokompatible Kunststoff Polyethylen ist.

13. Implantat nach einem der Ansprüche 1 - 12, **dadurch gekennzeichnet, dass** es mit Hydroxylapatit oder Tricalciumphosphat (TCP) beschichtet ist.

## Claims

1. Intervertebral implant comprising a flattened shaped hollow element (1), having an outer surface (2) that includes a flattened upper bone-contact face (3), a flattened lower bone-contact face (4) and a lateral face (5), whereby the upper and lower bone-contact faces (3;4) are elastically compressible towards the interior space (6) of the hollow element (1),
**characterized in that**
a) the hollow element (1) is shaped as a hollow cylinder, hollow truncated cone, hollow truncated pyramid or hollow truncated wedge;
b) the outer surface (2) of the hollow element (1) includes a second lateral face (5); and
c) upon application of a force of pressure the upper and lower bone-contact faces (3;4) are compressible towards each other such that the maximum distance H between the upper and lower bone-contact faces (3;4) is reducible between 0,5 - 5,0 mm.

2. Implant according to claim 1, **characterized in that** the maximum distance H between the upper and lower bone-contact faces (3;4) is reducible between 1 - 3 mm.

3. Implant according to claim 1 or 2, **characterized in that** the maximum distance H is reducible by 30% to 40% upon application of a force of pressure of 12 Newton onto the upper and lower bone-contact faces (3;4).

4. Implant according to claim 3, **characterized in that** the maximum distance H is reducible by 20% to 25% upon application of a force of pressure of 12 Newton onto the upper and lower bone-contact faces (3;4).

5. Implant according to one of the claims 1 to 4, **characterized in that** within the interior space (6) of the hollow element (1) an engagement lip (10) is provided, which is engageable within a interlock means (11) such that compression of the hollow element (1) is prevented.

6. Implant according to one of the claims 1 - 5, **characterized in that** the lateral faces (5) are provided with elongated apertures.

7. Implant according to one of the claims 1 - 6, **characterized in that** the upper bone-contact face (3) and/or the lower bone-contact face (4) are provided with a three-dimensional structure (7).

8. Implant according to claim 7, **characterized in that** the three-dimensional structure (7) is realised by means of longitudinal grooves.

9. Implant according to one of the claims 1 - 8, **characterized in that** the upper bone-contact face (3) and/or the lower bone-contact face (4) are provided with perforations (8).

10. Implant according to one of the claims 1 - 9, **characterized in that** the upper bone-contact face (3) and/or the lower bone-contact face (4) are provided with one or a plurality of central grooves (9).

11. Implant according to one of the claims 1 - 10, **characterized in that** it is made of a biocompatible plastic material.

12. Implant according to claim 11, **characterized in that** the biocompatible plastic material is polyethylene.

13. Implant according to one of the claims 1 - 12, **characterized in that** it is coated with hydroxylapatite or tricalciumphosphate (TCP).

## Revendications

1. Implant intervertébral constitué d'un corps profilé creux aplati (1) dont la surface d'enveloppe (2) est constituée d'une surface supérieure aplatie de contact avec l'os (3), d'une surface inférieure aplatie de contact avec l'os (4) et d'une surface latérale (5), la surface supérieure et la surface inférieure de contact avec l'os (3; 4) pouvant être comprimées élastiquement par rapport à l'espace intérieur (6) du corps profilé creux (11),
**caractérisé en ce que**
a) le corps profilé creux (1) est configuré sous forme de cylindre creux, de tronc de cône creux, de tronc de pyramide creux ou de troncs de dièdre creux ;
b) la surface d'enveloppe (2) du corps profilé creux (1) présente une deuxième surface latérale (5) ; et
c) lors de l'application d'une force de poussée, la surface supérieure et la surface inférieure de contact avec l'os (3 ; 4) peuvent être comprimées l'une par rapport à l'autre de telle sorte que la distance maximale H entre la surface supérieure et la surface inférieure de contact avec l'os (3 ; 4) puisse être diminuée entre 0,5 et 5,0 mm.

2. Implant selon la revendication 1, **caractérisé en ce que** la distance maximale H entre la surface supérieure et la surface inférieure de contact avec l'os (3 ; 4) peut être diminuée entre 1 et 3 mm.

3. Implant selon la revendication 1 ou 2, **caractérisé en ce que** lors de l'application d'une force de poussée de 12 Newton sur la surface supérieure et la surface inférieure de contact avec l'os (3 ; 4), la distance maximale H peut être réduite de 15 % à 30 %.

4. Implant selon la revendication 3, **caractérisé en ce que** lors de l'application une force de poussée de 12 Newton sur la surface supérieure et la surface inférieure de contact avec l'os (3 ; 4), la distance maximale H peut être réduite de 20 % à 25 %.

5. Implant selon l'une des revendications 1 à 4, **caractérisé en ce que** dans l'espace intérieur (6) du corps profilé creux (1) est prévue une lèvre d'accrochage élastique (10) qui peut être encliquetée dans un arrêt (11) en supprimant la compressibilité du corps profilé creux (1).

6. Implant selon l'une des revendications 1 à 5, **caractérisé en ce que** les surfaces latérales (5) sont dotées d'évidements en forme de trous oblongs (12).

7. Implant selon l'une des revendications 1 à 6, **caractérisé en ce que** la surface supérieure de contact avec l'os (3) et/ou la surface inférieure de contact avec l'os (4) sont dotées d'une structuration tridimensionnelle (7).

8. Implant selon la revendication 7, **caractérisé en ce que** la structuration tridimensionnelle (7) est configurée sous la forme de rainures longitudinales.

9. Implant selon l'une des revendications 1 à 8, **caractérisé en ce que** la surface supérieure de contact avec l'os (3) et/ou la surface inférieure de contact avec l'os (4) sont dotées de perforations (8).

10. Implant selon l'une des revendications 1 à 9, **caractérisé en ce que** la surface supérieure de contact avec l'os (3) et/ou la surface inférieure de contact avec l'os (4) présentent une ou plusieurs rainures centrales (9).

11. Implant selon l'une des revendications 1 à 10, **caractérisé en ce qu'**il est réalisé en une matière synthétique biocompatible.

12. Implant selon la revendication 11, **caractérisé en ce que** la matière synthétique biocompatible est le polyéthylène.

13. Implant selon l'une des revendications 1 à 12, **caractérisé en ce qu'**il est revêtu d'hydroxyapatite ou de phosphate tricalcique (TCP).
